# EUROPEAN PATENT SPECIFICATION

(11) **EP 3 021 919 B1**
(45) Date of publication and mention of the grant of the patent: **04.10.2017**
(21) Application number: 13744944.3
(22) Date of filing: 16.07.2013
(51) Int. Cl.: A61M 15/00

(54) **POWDER INHALER**
PULVERINHALATOR
INHALATEUR À POUDRE SÈCHE

(43) Date of publication of application: 25.05.2016
(73) Proprietor: EMPHASYS IMPORTADORA, EXPORTADORA E DISTRIBUIDORA DE EMBALAGENS LTDA., 18540-000 PORTO FELIZ, SP (BR)
(72) Inventor: ESTEVE, Victor, 04545-006 São Paulo (BR); ZEMBROD, Eric, 18550-000 Boituva - SP (BR)
(74) Representative: Manresa Val, Manuel
(86) International application number: PCT/BR2013/000254
(87) International publication number: WO 2015/006838

(56) References cited:
- EP-A2- 2 357 015
- WO-A1-97/27892
- WO-A1-2013/016784
- US-A1- 2004 187 867
- US-A1- 2006 169 280

## Description

### Field of the Invention

This Invention addresses technical and functional enhancements introduced to an inhaler developed particularly for use with inhalable powdered medications prepared in capsules containing a single dose. This device is widely used for delivering inhale medications for the treatment of respiratory diseases, where each capsule holds a single dose.

More particularly, the present invention consists in an improvement that can be introduced in different inhalers which work based on inhalable powdered medications prepared in capsules containing a single dose, such as the one disclosed in the document WO2013/016787 A1 published on 07/02/2013. The changes are concentrated in the suction means of the airflow, including means for aeration, or false air inlet, concurrently with the air inlet during inhalation, with objective of setting pre-measured mixtures of air and inhaled powdered medication at certain inhalation resistances, affecting de-agglomeration of the formulation and its fine particle fraction FPF, or inhalable fraction, which wouldn't de possible with state-of-the art capsule based dry powder inhalers.

### State of the Art

As described in WO2013/016787, a variety of inhalers available for delivering powdered medications prepared in type of single capsules, multidose-reservoir or in form of trip-blisters with independent recesses containing doses, such as those disclosed in the following documents:
BRPI0501263; BRPI0415711, BRPI0710078, CA2391466, DE19637125, EP0406893, EP0666085, EP0911047, EP1270034, EP1350532, US3906950, US3991761, US5372128, US7284552, US7870856, WO9727892, WO2004035121, VV02004052435, WO2005044353, VV02005113042, VV02006051300, WO2007116002, and GB2151491 A.

In addition, the document US2006/016982 should be mentioned. Device disclosed in US2006/016982 comprises an inhalation device for transpulmonary administration which is actuated by inhalation and having a primary and auxiliary flow path of air. The primary air flow path passes through the powder to entrain it while the auxiliary flow path does not pass through the powder but runs in parallel and is mixed with the primary flow path upon inhalation in the proximity of the mouthpiece.

Capsule based inhalers are small, easy to handle and economic to be manufactured since they require fewer components and allow reutilization; therefore they are candidates for delivering generic formulations. Such devices generally present their respective innovative characteristics, although in most cases these characteristics are incorporated in basic parts, such as the compartment for inserting a single dose capsule containing powdered medication; means for perforating the capsule at two opposite points at least for creating small openings in form of outlets for releasing the powdered medication; structure for inflow of air created by breath activate aspiration; channeling of this air flow through a de-agglomeration chamber; connected to a capsule compartment for releasing the formulation mixture with air; and a mouthpiece structure for inhalation the air flow with medication.

As appears, single dose capsule inhalers has practically resulted in the establishment of a standard device which allows the powdered medication to be inhaled efficiently from a capsule, for different types of treatments related to respiratory diseases, many of them chronic and widespread, including asthma, bronchitis and Chronic Obstructive Pulmonary Disease (COPO). Rigid gelatin or HPMC capsules containing excipients and micronized active substances, either individually or in combinations, are used in such inhalers.

Thus, known devices for inhaling powdered medications prepared in capsules work with the capsules held in a receptacle as taught for example in documents EP1350532A2 and US3906950A; or loose in a de-agglomeration chamber which dimensions are only large enough to allow subjecting the capsule to specific movements, as taught for example in documents BRPI0415711A, BRP10501263A,BRPI0710078, EP0911047A1, US5048514A, WO2004052435A1, WO2007116002A1, WO2007116002A1, WO2006051300A1, CA2391466C, EP1270034A2, US3991761A, US728455282, and WO2005113042A1. These movements may occur with the capsule in a vertical or in the horizontal position.

In vertical position, the capsule longitudinal axis is in vertical position and, consequently its chamber is defined by a cylindrical area with a diameter sized to hold the capsule in the vertical position, as taught in documents: BRP10415711A, EP0911047A1, EP0491426A1, US5048514A, US3906950A, WO2004052435A1, WO2005044353A1, WO2006051300A1 and WO2007116002A1.

In the horizontal position, the capsule longitudinal axis is positioned horizontally in a chamber also defined by a cylindrical area with a diameter larger than the length of the capsule in order to hold it in a horizontal position, such as taught for example, in documents: BRP10501263A, BRPI0710078, CA2391466C, EP1270034A2, US3991761 A, US7284552B2 and WO2005113042A1.

In both constructive cases, meaning with the capsule working in vertical or horizontal position, the capsule holding the dose it is subject to circular movements around its longitudinal axis, and rectilinear movements in random directions, knocking against the walls of its de-agglomeration chamber.

On the other hand, devices with the capsule in a horizontal position, such capsule is subject to rotation movements like a propeller. It is noted that the movements of the capsule are an important and decisive factor for encouraging air circulation and breaking down the powder for releasing the dose during inhalation. These inhalers use different ways for opening the capsule, or breaking through the capsule membrane, or piercing the capsule at opposite ends, in order to allow the air to flow into such capsule and release the formulation.

Each inhaler is embodied with intrinsic physical constructive characteristics that together with the formulation shape the pulmonary deposition and dose releasing profile. Dry powdered formulations prepared in capsules consists mainly of a blend of lactose and micronized active substances that must be broken-down during inhalation in order to allow the release of the dose with an adequate percentage of fine particle fraction, or breathable fraction (considered particles smaller than 4,6µ micra). Within lung deposition, the breathable fraction is the percentage of the formulation reaching the lower portion of lungs, a relevant parameter for determination of the product therapeutic efficacy. For capsule based dry powder inhalers, this breathable percentage may vary from 15% to 50% of the emitted dose, however, independent on the inhaler type, such percentage values may differ based formulation and substance, and can de considered adequate based of the parameters found in registered products, acknowledged as Reference Leading Drugs (RLD).

One of the parameters for analyzing the performance of a dry powder inhaler is through its inhalation resistance, which determines the volume of air which enters the inhaler at a given inhalation capacity, which can be measured in L/min. (liters per minute). This volumetric flow may be calculated by using the flow resistance at a specific inhalation pressure, measured in Kilopascal-kPa (or pressure drop). A 4kPa parameter is normally addressed as it is given by the European pharmacopeia and the USP as the inhalation pressure parameter (pressure drop) for adjusting analytical equipments for dry powder inhalers in-vitro formulation assays.

In order to ensure adequate release of certain formulations, it is preferable to use inhalers which allow constructive means for influencing lung deposition profile, breathable fraction, and the inhalation resistance.

Usually, to adjust the breathable fraction, a carrier can be used, as for example, inhalable lactase with different levels of micronization to thereby reduce or increase the breathable fraction percentage, in order to achieve a certain adequate parameter in-vitro result. However, adjustment of fine breathable fraction via carrier micronization has limitations which may restrict the use of capsule based inhalers (single dose capsule) for administration of certain drug medications in view of the limitation to obtain a satisfactory lung deposition profile only via formulation.

The lung stages can be simulated by using equipments that identify the dimension of the particles, sucking the inhalable powder in determined air flow, through various filter patterns to establish the percentage of particle retention in different stages each identified by deposition of determined size of particles in form of a cascade. Such equipments, also known as "cascade impactor", can be operated in distinct inhalation flows to simulate the lung deposition profile for different lung capacities (L/min). These apparatus enable to collect the exact amount of lactase and active substance in each stage, simulating the human inhalation in stages, such as mouth, throat, trachea and lungs; represented in various stages of distribution; the early stages representing the deposition in the upper parts of the lung, and the final stages representing the deposition in the lower part of the lung.

Although there are available various grades of micronized lactase, it is not always technically possible to achieve the adequate deposition result in all lung stages, especially in lower stages where percentages of active substances are quantitatively smaller, but not less relevant to determine product efficacy. Another limiting aspect relates to certain quantitative standards for carriers and active substance in each stage of lung deposition. If the construction of a determined inhaler does not present aerodynamic means to enable achievement of a desired lung deposition profile, the quantity of micronized lactose and drug substance can be eventually above or below the desired pattern preventing the release of formulations qualitatively and quantitatively desirable.

Inhalers such as those disclosed in BRPI0501263, BRPI0415711, BRPI0710078, CA2391466, DE19637125, EP0406893, EP0666085, EP0911047, EP1270034, EP1350532, EP2010258, BRPI0710078, US3906950, US3991761, US5048514, US5372128, US7284552, US7870856, WO9727892, WO2004035121, WO2004052435, WO2005044353, WO2005113042, WO2006051300, WO2007116002 and GB2151491A describes individual constructive characteristics, not elaborating on specific means to influence or regulate the releasing of the formulation and its deposition profile, limiting their effectiveness for the releasing of formulations with certain parameters.

The pressure or inhalation resistance is other relevant aspect but not decisive for obtaining a determined lung deposition profile.

Therefore, the objective to solve the problem should take into consideration a constructive feature in a powder inhaler which capsule spins in its horizontal axis, which can influence different aerodynamic aspects contributing to enable preparation of formulations to achieve adequate deposition of active substance in lung stages with the aimed inhalation resistance.

The formulation release profile can also influenced by the manner and site where the capsule is opened. Normally, dry powder inhalers use needles or pins to pierce the capsule at its ends. This is designed to ensure that the air flow also penetrates the capsule through a vortex, encouraging the creation of the spray in the de-agglomeration chamber housing the capsule, resulting in a mixture of the air with the inhalant substance that flows through the mouthpiece, and from there to the lungs. The breakdown chamber, which houses the capsule, is therefore decisive for the drug release profile, and part of the set responsible for inhalation resistance.

WO2013/016787 aims specific solutions to achieve above objectives, and therefore, the inhaler has been improved in its air powder mixture chamber and adjacencies, more specifically at the air flow outlet and route. A passage was introduced in the roof of this de-agglomeration chamber with specific geometry, carefully dimensioned, normally rectangular or circular, which constitutes an outlet for the inhalant, with the length of this outlet also being preferably equal to the length of the cylindrical part of the capsule (except rims) and its width is approximately 1/3 or less than its diameter. Logically, this opening is fitted with a sieve-like structure at an appropriate mesh, in order to retain possible particles whose dimensions are not appropriate for inhalation.

Thus, during inhalation process, the capsule is subject to a variety of rotating and rectilinear movements in the vertical or horizontal positions, consequently leading to the affirmation that, as the air flow enters the inhaler, the capsule rotates horizontally like a propeller and is concomitantly moved outwards and downwards, hitting the bottom and roof of its chamber. When it is up against the roof, a specific effect occurs, because at a given movement, the capsule and the outlet are aligned, thus producing a valve-like effect, meaning that the capsule is practically sucked into the rectangular outlet and at this moment the airflow is reduced for a fraction of a second, due to the rotation of the capsule, thus defining a new standard of functioning through which the air outlet from the capsule chamber is blocked intermittently during inhalation. These sudden blockages in fact generate additional forces with micro-collisions of the capsules against the inner walls of the chamber, producing other effects that cause the powder in the capsule to be subject to bursts that move the clumped powder in directions opposite to the centrifugal and gravitational forces at its ends, fostering breakdown and release with greater efficiency normally achieved merely through the vortex effects in the chamber. In this case, the brief intermittent blockages of air occur when the displacement of the capsule in the air flow forces it up against the air outlet from the chamber, with both longitudinal axes aligned.

Thus, WO2013/016787 presents an alternative form for the release of inhalable powdered formulations with a more efficient pulmonary deposition profile for an inhaler that functions with the capsule in a horizontal position.

On the other hand, WO2013/016787 describes an improvement in construction for a version of a powder inhaler that functions with a capsule subject to a horizontal rotating movement, which offers means for achieving an adequate powder release profile at high inhalation resistance, in contrast to the state-of-the-art model for dry powder inhalers that work with the capsule in a horizontal position and with lower inhalatory pressure.

There is no doubt that WO2013/016787 discloses a more flexible operation, particularly with regard to the desired percentage of formulation that reaches the lower part of the lung, region with the greatest importance for the effectiveness of the medication. However, after further tests of in-vitro characterization with newly invented test model, it was verified that would be possible to expand the flexibility of the inhaler adding means to influence the release profile and inhalation resistance, concomitantly with a significant improvement enabling development of powder formulations that can reach certain percentages of active substances in the respective lung stages.

### Objective of the Invention

Provide constructive means to influence air flow distribution and inhalation pressure to allow rebalancing air route through aerodynamic construction of a dry powder inhaler, type which capsule containing dose works in horizontal position, with objective to achieve desirable results in the lung deposition stages, mainly lower lung stages where percentages of active substances are quantitatively smaller but decisive for clinic effectiveness of the formulation.

Proposed means by the present invention consists to provide false openings or entries that allow establishing additional air flow concurrently with the main air flow, which conducts the medication and, through a de-agglomeration chamber and mouthpiece portion, such additional flow is mixed with the main inhalation flow, providing a new parameter for influencing the internal aerodynamic balance on the device and pattern of effectiveness for formulation de-agglomeration given by said "aeration effect on the formulation" which allows considerably to influence the medication release profile, contributing to achieve adequate percentages of active substance deposition within the different lung stages, which could not be achieved only via carrier micronization.

### Description of the drawings

For a better understanding of this invention, a detailed description thereof is presented below, referenced to the appended drawings:
**FIGURES 1 and 2** represent isometric views showing the inhaler completely closed;
**FIGURES 3 and 4** show, respectively, a view in anterior elevation and a superior view, where are also indicated the cross sections A-A and B-B;
**FIGURE 5** illustrates an elevation view showing the inhaler in accordance with the transversal cross section "B-B" indicated in the figure 4;
**FIGURE 6** is another view in elevation showing the inhaler in accordance with the transversal cross section "A-A" indicated I figure 3;

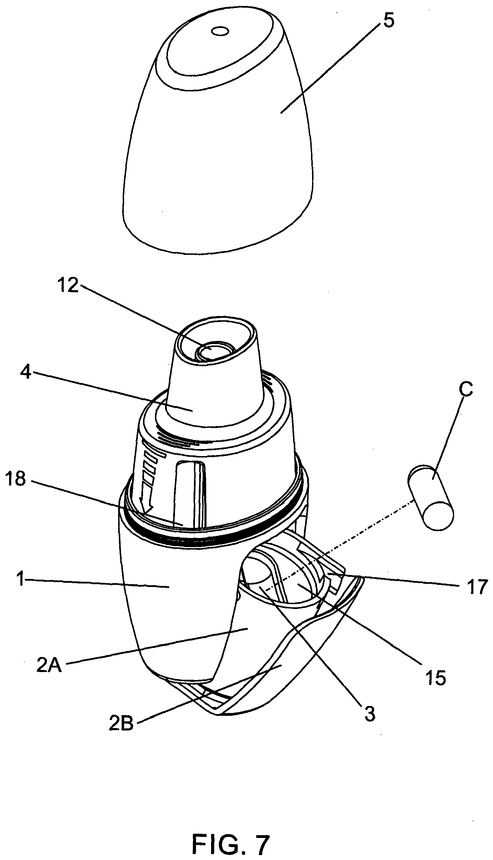

**FIGURE 7** represents an enlarged isometric view showing the inhaler with the lid displaced and the capsule receptacle in position to receive said capsule;
**FIGURE 8** shows a set of views illustrating the functioning of the set; and the
**FIGURE 9** shows a plan view highlighting the operation of the present invention;

### Detailed description of the invention

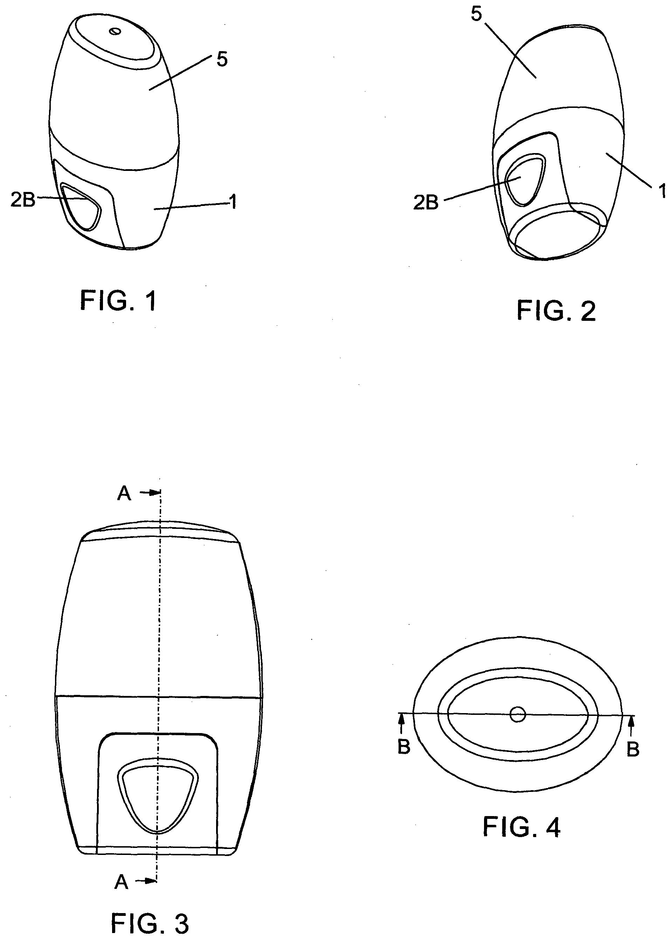

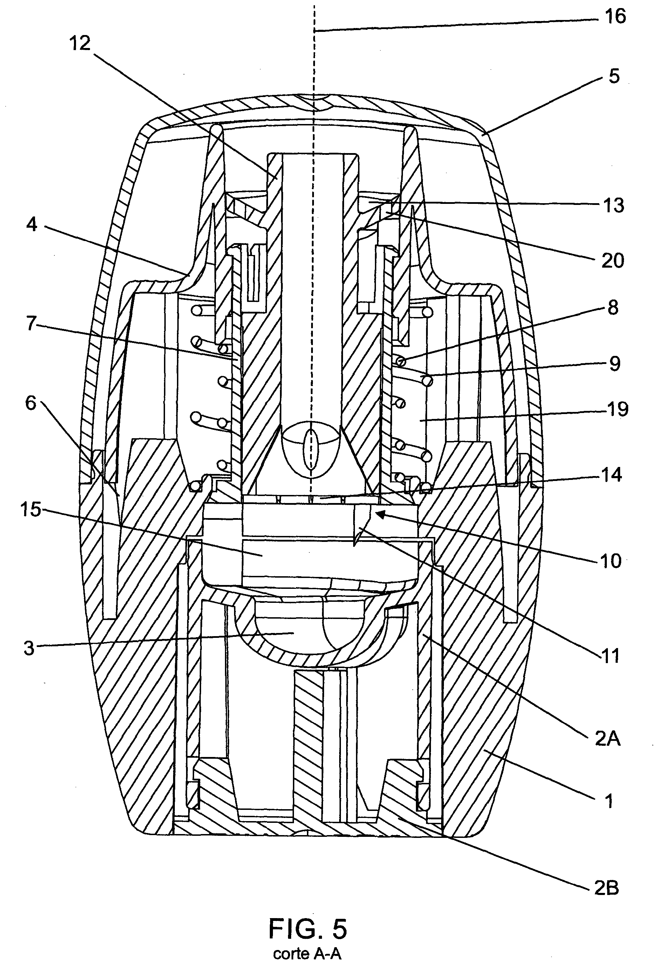

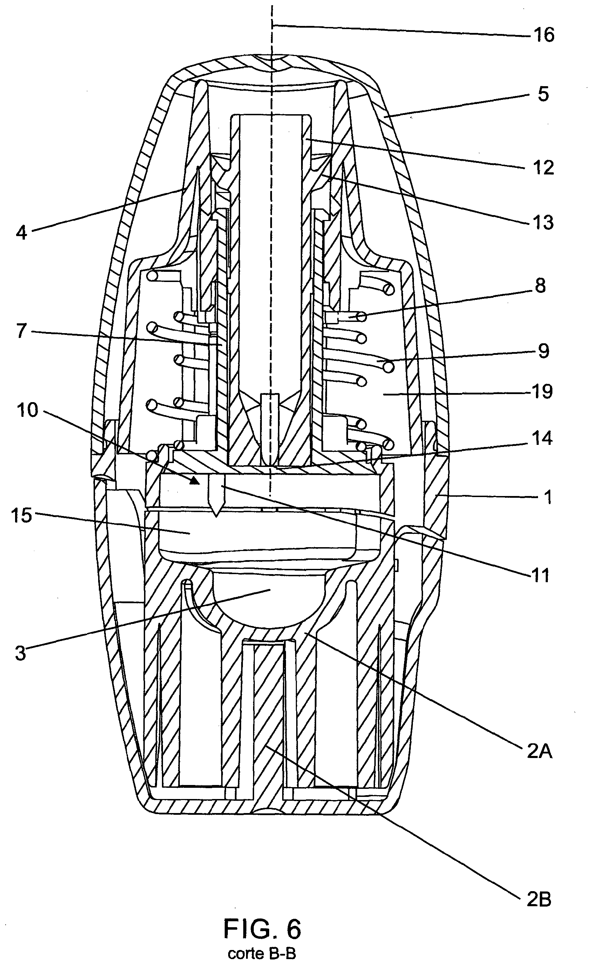

In compliance with these illustrations and their details, more specifically the figures 1 to 6, this POWDER INHALER is applicable to a type that has been developed especially for use solely with inhalable powdered medications prepared in capsules containing a single dose which works with the capsule rotating in horizontal position, such as that taught in documents WO2007/098870 (BRPI0710078) and WO2013/016787, consisting of:
- base housing (1) with a cross section that is normally oval and completely hollow;
- a snap-in capsule receptacle (2A) mounted pivotally within the lower half part of the base housing (1) together with a lid (28) and with sufficient means to be pivoted outwards and expose its slot-in cradle (3) capsule housing (C) containing powdered inhalant medication, and means for such capsule receptacle to return to the initial position aligned with the longitudinal axis of the base housing (1);

- a moveable mouthpiece (4) affixed on the upper part of the base housing (1), with this mouthpiece having a cap on the outside (5), while on the lower side it has lateral stems (6) slidably coupled on a vertical guide (7), something tubular, where said mouthpiece (4) can be moved vertically downwards or outwards in combination with helical springs (8 and 9), in which the first downward movement is realized by manual pressure that exceeds the strength of the springs, and the return movement upwards is due to the force of this helical springs (8 and 9);
- a perforation device (10) for opening the capsule (C), affixed to the inner side of the mouthpiece (4), which device, in addition to being moved together with the mouthpiece (4), also has means consisting of a pair of vertical needles (11) whose lower sharp points are positioned to radially perforate the ends of the capsule (C) forming small openings for the outflow of the powdered medication;
- a flow guide tube (12), centralized and housed on the inner side of the mouthpiece (4) and the guide (7), with the upper edge ending practically within said mouthpiece (4), adjusting the latter by a flange (13), while the lower part has a sieve-like structure (14) that, in its turn, forms the roof of the de-agglomeration chamber (15) formed above the housing (3) of the capsule (C), consequently, between said de-agglomeration chamber (15) and the upper edge of the mouthpiece (4) a vertical passage (16) is formed for the air flow and the medication;

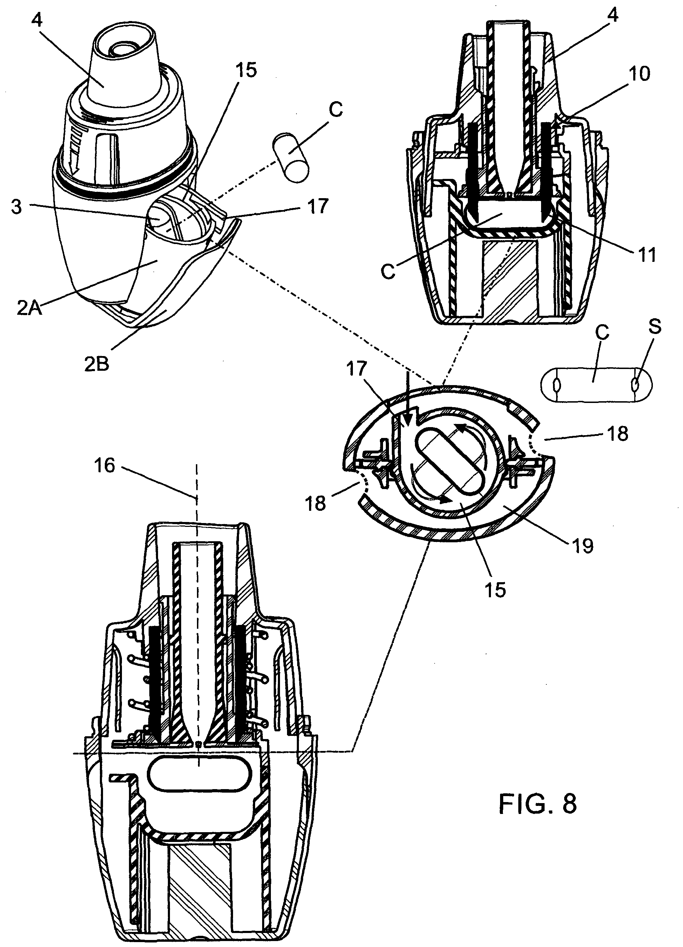

The above-mentioned mouthpiece (4) also has means to establish an inward air flow from outside and is hollow in order to do so, forming that vertical passage (16) for the inhalant, whose lower end is connected to the capsule receptacle (2A) which, above the slot-in cradle (3), has a wider portion that constitutes the de-agglomeration chamber (15), cylindrical, with a diameter slightly larger than the length of the capsule (C), and also has a tangential secondary air intake point (17) positioned between the walls of the capsule receptacle (2A) and the base housing (1) which in turn has one or two primary air intake points (18), with a pocket (19) formed between them which improves the stability of the air flow created when the patient breathes in during the inhalation process, being this operation illustrated in figure 8, where may be noted that the inhalation process begins when the snap-in capsule receptacle (2A) is packed with a capsule (C) containing powdered medication. The capsule (C) slots smoothly into the cradle (3), avoiding movement. When the capsule receptacle (2A) is snapped back into its original position (closed), the capsule remains in a stable position so that the opening device (10) can be brought into action by pressing the mouthpiece (4) through its surrounding shoulder, while the needles (11) move downwards and radially perforate the ends of the capsule (C), forming openings (S) for the outflow of the powdered medication, which occurs only when the user breaths in through the mouthpiece (4). Such aspiration results in an airflow that runs through the primary intake point (18), the air pocket (19) and the secondary intake point (17) tangentially reaching the interior of the de-agglomeration chamber (15), where the vortex effect causes an outflow from the capsule (C) in its cradle, (3) at which time it starts to spin and, due to the restrictions of the de-agglomeration chamber (15), during this spinning movement it nevertheless remains in a horizontal position. The capsule movements allow the outflow of the powder that it holds, allowing the air/powder mixture to be formed by the vortex in the de-agglomeration chamber (15), which can flow out along the conduit (16) and reach the lungs of the user.

Laboratory tests for in-vitro characterization of lung stages deposition with new constructive features has proven that would be possible to expand the flexibility of such kind of inhalers by adding means to influence the release profile and inhalation resistance, concurrently with significant improvement in the final result to certain deposition profiles, aiming elaboration of powder formulations with adequate percentages of active substance reaching respective lung stages.

Changes along the passage (16) can significantly influence aerodynamic flow and inhalation pressure, concurrently, such influence also collaborate to assist to reach the aimed results in the lung deposition stages, especially in lower stages where percentages of active substances are quantitatively smaller.

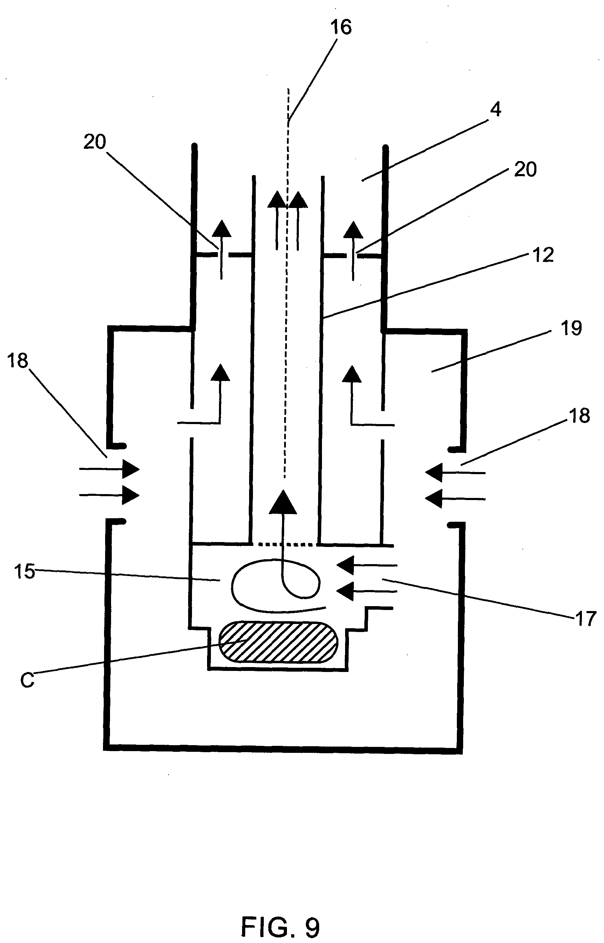

Thus, as also illustrated in figure 9, the present invention has the primary objective to allow changes in the balance parameters for driving the air flow through the inhaler internal aerodynamics during its enhancement with the medication and, consequently, passing through the de-agglomeration chamber (15), and more precisely the changes in the characteristics of the air flow that occur after the air moves into the inhaler by the primary air inlets (18) and its distribution along the inlet (17) of the de-agglomeration chamber (15) and the inlet channels to the inner part of the mouthpiece (4) and, therefore, the present invention is characterized by the fact that it includes one or more false air inlets (20), apart from the passage (16) and that provide an additional or secondary air flow of aspiration that is formed with the air captured in the air pocket (19) and that moves into the inhaler by the primary air inlets (18). This secondary air flow rises concurrently with the main air flow that conduct the medication and, in the mouthpiece portion (4), the additional flow is mixed with the main inhalation flow, providing an aeration effect that influence the release profile and mixing of medication, contributing the achieve certain percentages of active substances in the lung deposition stages. The passages (20) provide a parallel air flow which is formed outside the flow guide tube (12) and also outside the de-agglomeration chamber (15), consequently, this flow does not conduct medication while it does not reach the inner part of the mouthpiece (4), and in its inner part such air flow is mixed with the air that is formed along the passage (16), which, if properly dimensionally calibrated, assists to balance the inhalation resistance (L/min) and lung deposition profile via fraction of fine particles. The passages (20) also collaborate to improve the stability of the air flow during inhalation, being possible to achieve certain lung deposition profiles, where the combination of such openings led a more flexible operation to the set, enabling to equalize inhalation resistance and release profile given by fine breathable fraction (smaller than 4,6µ micra) in according to the efficiency parameters provided.

## Claims

1. **POWDER INHALER,** consisting of:
- a dry powder inhaler for use with capsule (C) which works in a horizontal position
- base housing (1) with upper and lower half part, wherein its cross section is oval and hollow;
- a capsule receptacle (2A) mounted pivotally within the lower half part of the base housing (1) together with a lid (2B) and means to be pivoted outwards and expose its cradle (3) for a capsule housing (C) containing powdered inhalant medication, and means for such capsule receptacle to return to the initial position aligned with the longitudinal axis of the base housing (1);
- a moveable mouthpiece (4) affixed on the upper part of the base housing (1), with this mouthpiece having a cap (5) on the outside, and having on the lower side of that moveable mouthpiece (4) lateral stems (6) slidably coupled to tubular vertical guide (7), wherein said mouthpiece (4) is movable downwardly or outwardly in combination with helical springs (8 and 9), in which the first movement is realized by manual pressure that exceeds the strength of the springs, and the return movement is due to the force of this helical springs (8 and 9);
- a perforation device (10) for opening the capsule (C), affixed to the inner side of the mouthpiece (4), wherein the perforation device (10) being moved together with the mouthpiece (4), also has means consisting of a pair of vertical needles (11) whose lower sharp points are positioned to radially perforate the ends of the capsule (C) forming small openings for the outflow of the powdered medication;
- a flow guide tube (12), centralized and housed on the inner side of the mouthpiece (4) and the guide (7), with the upper edge ending within said mouthpiece (4), adjusted by a flange (13), while the lower part has a sieve-like structure (14) forming the roof of a de-agglomeration chamber (15) formed above the cradle (3) for the capsule (C), wherein between said de-agglomeration chamber (15) and the upper edge of the mouthpiece (4) a vertical passage (16) is formed for the main air flow and the medication; and said de-agglomeration chamber (15) has a tangential secondary air intake point (17) positioned between the walls of the capsule receptacle (2A) and the base housing (1) which in turn has one or two primary air intake points (18), with a air pocket (19);
**characterized by** the fact that that it includes one or more false air passage (20), apart from the passage (16) and that provide an additional air flow of aspiration that is formed with the air captured in the air pocket (19) and that moves into the inhaler by the primary air inlets (18), this additional air flow rises concurrently with the main air flow that conducts the medication from the de-agglomeration chamber (15) and, in the mouthpiece portion (4), the additional flow is mixed with the main inhalation flow.

## Patentansprüche

1. **PULVERINHALATOR,** bestehend aus:
- einem Trockenpulverinhalator zur Verwendung mit einer Kapsel (C), die in einer horizontalen Position arbeitet
- einem Grundgehäuse (1) mit einer oberen und unteren Hälfte, wobei sein Querschnitt oval und hohl ist;
- einer Kapselaufnahme (2A), die schwenkbar in der unteren Hälfte des Grundgehäuses (1) montiert ist, zusammen mit einem Deckel (2B) und Mitteln, um nach außen geklappt zu werden und seine Schale (3) für ein Kapselgehäuse (C), das ein pulverförmiges Inhalationsmedikament enthält, freizulegen, und mit Mitteln, damit eine solche Kapselaufnahme in die Ausgangsposition ausgerichtet mit der Längsachse des Grundgehäuses (1) zurückkehrt;
- einem beweglichen Mundstück (4), das am Oberteil des Grundgehäuses (1) angebracht ist, wobei dieses Mundstück eine Kappe (5) an der Außenseite hat, und an der Unterseite dieses beweglichen Mundstücks (4) seitliche Schäfte (6) hat, die verschiebbar an die rohrförmige vertikale Führung (7) gekoppelt sind, wobei das Mundstück (4) abwärts oder auswärts in Kombination mit Spiralfedern (8 und 9) beweglich ist, wobei die erste Bewegung durch manuellen Druck durchgeführt wird, der die Kraft der Federn überwindet, und die Rückstellbewegung auf die Kraft dieser Spiralfedern (8 und 9) zurückzuführen ist;
- einer Perforationsvorrichtung (10) zum Öffnen der Kapsel (C), die an der Innenseite des Mundstücks (4) angebracht ist, wobei die Perforationsvorrichtung (10), die zusammen mit dem Mundstück (4) bewegt wird, auch Mittel hat, bestehend aus einem Paar vertikaler Nadeln (11) deren untere scharfen Punkte positioniert sind, um die Enden der Kapsel (C) radial zu perforieren und kleine Öffnungen zum Auslaufen des pulverförmigen Medikaments zu bilden;
- einer Strömungsführungsleitung (12), die an der Innenseite des Mundstücks (4) und der Führung (7) zentralisiert und aufgenommen ist, wobei der obere Rand angepasst durch einen Flansch (13) mit dem Mundstück (4) endet, wobei das untere Teil eine siebähnliche Struktur (14) hat, die das Dach einer Desagglomerationskammer (15) bildet, die über der Schale (3) für die Kapsel (C) gebildet ist, wobei zwischen der Desagglomerationskammer (15) und dem oberen Rand des Mundstücks (4) ein vertikaler Durchgang (16) für die Hauptluftströmung und das Medikament gebildet ist; und wobei die Desagglomerationskammer (15) einen tangentialen sekundären Lufteinlasspunkt (17) hat, der zwischen den Wänden der Kapselaufnahme (2A) und dem Grundgehäuse (1) positioniert ist, das wiederum einen oder zwei primäre Lufteinlasspunkte (18) mit einer Lufttasche (19) hat;
**gekennzeichnet durch** den Umstand, dass sie einen oder mehrere Falschluftdurchgänge (20) zusätzlich zum Durchgang (16) hat und eine zusätzliche Ansaugluftströmung bereitstellt, die mit der gefangenen Luft in der Lufttasche (19) gebildet wird, und die sich durch die primären Lufteinlässe (18) in den Inhalator bewegt, wobei diese zusätzliche Luftströmung gleichzeitig mit der Hauptluftströmung ansteigt, die das Medikament aus der Desagglomerationskammer (15) leitet, und im Mundstückabschnitt (4) die zusätzliche Strömung mit der Hauptinhalationsströmung gemischt wird.

## Revendications

1. **INHALATEUR DE POUDRE** comprenant:
- inhalateur de poudre sèche à utiliser avec une capsule (C) fonctionnant en position horizontale;
- carcasse de base (1) à moitié supérieure et inférieure, où la section transversale est ovale et creuse;
- un réceptacle à capsule (2A) monté en rotation dans la moitié inférieure de la carcasse de base (1) avec un couvercle (2B) et un moyen lui permettant de pivoter vers l'extérieur et de faire apparaître le support (3) pour loger la capsule (C) contenant un médicament en poudre à inhaler, et un moyen pour que ledit réceptacle à capsule revienne à la position initiale en ligne avec l'axe longitudinal de la carcasse de base (1);
- un embout buccal mobile (4) fixé à la partie supérieure de la carcasse de base (1), ledit embout buccal ayant un bouchon (5) à l'extérieur, et sur le côté inférieur de cet embout buccal mobile (4) des tiges latérales (6) accouplées en glissant sur le guide vertical tubulaire (7), où ledit embout buccal (4) se déplace vers le bas ou vers l'extérieur en combinaison avec des ressorts hélicoïdaux (8 et 9), où se produit le premier mouvement par pression manuelle supérieure à la force des ressorts, et le mouvement de retour est dû à la force de ces ressorts hélicoïdaux (8 et 9);
- dispositif perforateur (10) pour ouvrir la capsule (C), fixé au côté interne de l'embout buccal (4), où le dispositif perforateur (10) étant déplacé avec l'embout buccal (4) a également un moyen comprenant deux aiguilles verticales (11) dont les pointes acérées inférieures sont positionnées de manière à perforer radialement les extrémités de la capsule (C) en formant de petites ouvertures pour laisser sortir le médicament en poudre;
- un tube de guidage (12), centralisé et logé sur le côté interne de l'embout buccal (4) et le guide (7), le bord supérieur terminant dans ledit embout buccal (4), ajusté par une collerette (13), alors que la structure de la partie inférieure fait fonction de sas (14) formant la partie supérieure de la chambre de désagglomération (15) formée au-dessus du support (3) de la capsule (C), où entre ladite chambre de désagglomération (15) et le bord supérieur de l'embout buccal (4) un passage vertical (16) est formé pour le flux d'air principal et le médicament ; et ladite chambre de désagglomération (15) a un point d'entrée d'air secondaire tangentiel (17) positionné entre les parois du réceptacle à capsule (2A) et la carcasse de base (1) qui a également un ou deux points d'entrée d'air primaire (18), avec une poche à air (19);
**caractérisé en ce qu'**il inclut un ou plus d'un faux passage d'air (20), en plus du passage (16) et qui fournit un flux d'air additionnel d'aspiration qui est formé avec l'air prisonnier dans la poche à air (19) et qui se déplace dans l'inhalateur par les entrées d'air primaire (18), ce flux d'air additionnel se déplace simultanément avec le flux d'air principal qui conduit le médicament depuis la chambre de désagglomération (15) et, dans la portion de l'embout buccal (4), le flux additionnel se mélange au flux d'inhalation principal.
